# EUROPEAN PATENT APPLICATION

(11) **EP 4 177 330 A1**
(43) Date of publication of application: **10.05.2023**
(21) Application number: 21832192.5
(22) Date of filing: 01.07.2021
(51) Int. Cl.: C12M 1/34

(54) **CELL RECOVERY DEVICE**

(30) Priority: 01.07.2020 JP 2020114086
(71) Applicant: Nepa Gene Co., Ltd., Ichikawa-shi, Chiba 272-0114 (JP)
(72) Inventor: AOKI, Daiichiro, Fujimino-shi, Saitama 356-0036 (JP); NAGAMUNE, Teruyuki, Kawagoe-shi, Saitama 350-0808 (JP); OHKI, Rieko, Tokyo 104-0045 (JP); HEIKE, Yuji, Tokyo 104-0045 (JP); YAMAHIRA, Shinya, Tokyo 104-0045 (JP); YAMAGUCHI, Satoshi, Tokyo 153-8904 (JP); NOGUCHI, Masao, Funabashi-shi, Chiba 273-0862 (JP); KURIHARA, Kinya, Ichikawa-shi, Chiba 272-0114 (JP); ATSUMI, Yusuke, Ichikawa-shi, Chiba 272-0114 (JP); NAKAMOTO, Kazuki, Ichikawa-shi, Chiba 272-0114 (JP); SUZUKI, Kosho, Ichikawa-shi, Chiba 272-0114 (JP)
(74) Representative: Plougmann Vingtoft a/s
(86) International application number: PCT/JP2021/024942
(87) International publication number: WO 2022/004842

(57) **Abstract**

Disclosed is a cell collection device with which switching and seeing microscope images with different magnifications can be attained in one-touch without rotating a revolver. The cell collection device includes an optical system to observe cells contained in a transparent vessel under magnification. The optical system includes a first optical system to observe the cells from upper side and a second optical system to observe the cells from lower side. The first optical system and the second optical system have different magnifications, and the optical axis of the first optical system and the optical axis of the second optical system are coincident.

## Description

### Technical Field

The present invention relates to a cell collection device primarily used for the purpose of life science research.

### Background Art

As a result of allowing various information of cells to be analyzed by appearance of a next generation sequence technology, it has been found that, characters of cell populations propagated from a single cell significantly vary over time even between the same cells, unlike the conventional concept. For example, even in the same cancer cellular species, there is a cell for which an anticancer drug at a certain concentration works and a cell for which the anticancer drug does not work. Therefore, it is impossible to efficiently develop an antibody drug and elucidate the diseases such as those in the regenerative medicine field, allergy and Alzheimer's disease, without analysis in an individual cell level, and in a current situation, a single cell analysis technology is indispensable.

In association with such a change in life science research, various devices (hereinafter referred to as "cell isolation devices") that separate a single cell have been available, and as a great trend, the devices can be classified into low-end products of a manual operation type with a small number of collected cells per unit time and high-end products of an automatic processing type with a large number of collected cells per unit time. However, the latter products account for the majority of the devices.

As described in the following patent literatures as examples, as features of high-end products, for example, there is a high-end product that emphasizes automatic detection of a target cell in an image or a high-end product that emphasizes devising a cell culture vessel for easy and reliable collection of a target cell. This allows almost fully automatic isolation of cells in a scale of several thousands in a short period of time based on, for example, fluorescence signals of the cells.

On the other hand, as a feature of low-end products, the low-end product basically has a configuration of combining a display for visually confirming a microscopic image of cells directly by a user, position adjustment means for manually seeking for a target cell, and a fine glass tube for physically aspirating the target cell. Although a large number of cells cannot be isolated in a short period of time, it is an advantage that a user can isolate cells while confirming the cells.

### Citation List

### Patent Literature

Patent Literature 1: JP 2016-000007 A
Patent Literature 2: JP 2017-108738 A
Patent Literature 3: JP 2014-132897 A

### Summary of Invention

### Technical Problem

The high-end products described above can collect a large number of cells in a short period of time, but there is no guarantee that the collected cells are the intended cells. In the low-end products, since the user performs confirmation and isolation while seeing a microscope image, a proportion of ensuring isolating the cells intended by the user increases. However, for observation at a magnification necessary to determine whether the cells are the target cells, a magnification of a lens needs to be increased up to around x20. Meanwhile, to seek for the cells, the field of view needs to be widened at a low magnification, and therefore a labor that the lens magnification needs to be frequently changed occurs. Furthermore, when a revolver of a microscope is rotated to change a magnification from a low magnification to a high magnification, since the field of view is slightly shifted, a case in which the cell found through observation at a low magnification needs to be sought again at a high magnification often occurs.

An object of the present invention is to provide a cell collection device that allows switching and seeing microscope images with different magnifications in one-touch without rotating a revolver.

### Solution to Problem

As a result of intensive research, the present inventors conceived that microscope images of different magnifications can be switched and seen in one-touch by containing cells to be observed in a transparent vessel; and providing an optical system for observing the cells from upper side and an optical system for observing the cells from lower side of the cells, such that the optical systems have different magnifications and that the optical axes of both optical systems are coincident, to complete the present invention.

That is, the present invention provides the following:
(1) A cell collection device comprising an optical system to observe cells contained in a transparent vessel under magnification, wherein the optical system comprises a first optical system to observe the cells from upper side and a second optical system to observe the cells from lower side, the first optical system and the second optical system have different magnifications, and wherein the optical axis of the first optical system and the optical axis of the second optical system are coincident.
(2) The cell collection device of (1), wherein the first optical system has a magnification lower than that of the second optical system.
(3) The cell collection device of (2), wherein the magnification of the first optical system is from x4 to x10, and the magnification of the second optical system is from x20 to x40.
(4) The cell collection device of any one of (1) to (3), wherein the first optical system comprises a first light source and the second optical system comprises a second light source, and wherein the first light source enables observation with the first optical system and the second optical system, and the second light source enables observation with the second optical system and the first optical system.
(5) The cell collection device of (4), wherein the first light source and the second light source are of different types.
(6) The cell collection device of any one of (1) to (5), comprising a cell-aspirating means including an electro-osmotic pump, wherein the cell-aspirating means comprises a capillary to aspirate a cell at the distal end of the electro-osmotic pump, and a siphon tube communicating with a culture medium of the cells at the proximal end of the electro-osmotic pump.
(7) The cell collection device of (6), comprising a two-dimensional stage for placing the vessel containing the cells, a first driving system for driving the first optical system and the cell-aspirating means, and a second driving system for driving the two-dimensional stage, wherein the first driving system and the second driving system are independent, and the first driving system has a resolution lower than that of the second driving system.
(8) Use of the cell collection device of any one of (1) to (7) for collecting a single cell.
(9) A method of collecting a single cell, the method comprising collecting a single cell from cells contained in the vessel of the cell collection device of any one of (1) to (7). Advantageous Effects of Invention

The cell collection device according to the present invention allows switching and seeing microscope images at different magnifications in one-touch.

### Brief Description of Drawings

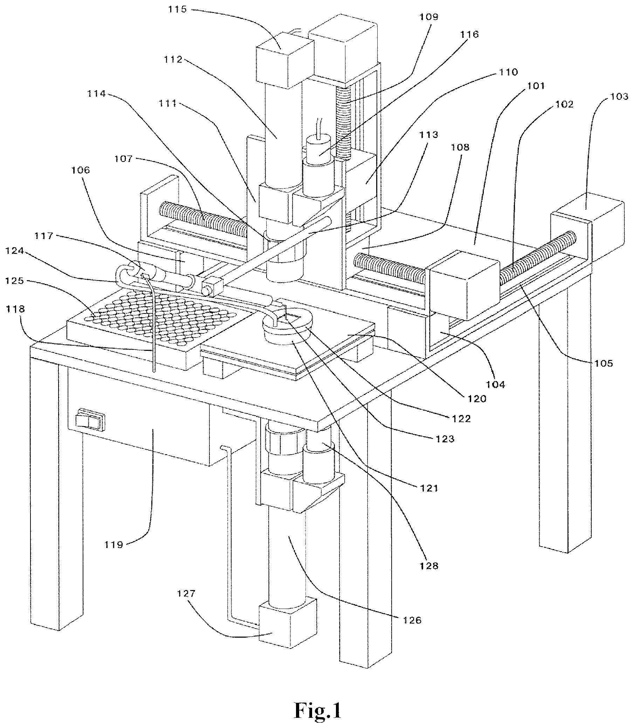

FIG. 1 is a schematic perspective view illustrating an appearance of a cell collection device according to one example of the present invention.

### Description of Embodiments

The cell collection device of the present invention includes an optical system (microscope) that magnifies cells contained in a transparent vessel for observation. The optical system comprises a first optical system and a second optical system. The first optical system is for observing the cells from upper side of the cells. The second optical system is for observing the cells from lower side of the cells. The first optical system and the second optical system have different magnifications, and the optical axes of the first optical system and the second optical system are coincident.

Since the optical axes of both optical systems are coincident, this configuration allows the cells to be always observed from upper side and lower side of the cells at different magnifications simultaneously. The microscope images at the two types of magnifications can be switched and seen in a monitor in one-touch without an operation such as rotation of a revolver. Without rotation of a revolver, the field of view of an observed image is not shifted at different magnifications.

The magnification of the first optical system is preferably lower than the magnification of the second optical system. This is because a liquid surface of a culture medium in which the cells are immersed shakes in some cases, and the influence of the shake of the liquid surface is small at a lower magnification. In the second optical system for observation from lower side, the cells are observed from the bottom surface side of the vessel. Therefore, the influence of the shake of a liquid surface is less likely to be received, thus not causing a problem in observation at high magnification. Usually, the magnification of the first optical system is approximately from x4 to x10. The magnification of the second optical system is approximately from x20 to x40. However, the magnifications are not necessarily limited thereto.

It is preferred that the first optical system comprise a first light source and the second optical system comprise a second light source. In this case, since the optical axes of both optical systems are coincident, the first light source allows observation with the first optical system and the second optical system, and the second light source allows observation with the second optical system and the first optical system. Both light sources may be the same light source, but the use of different types of light sources is advantageous to ensure observation using different types of light sources. For example, it is possible to perform usual observation with a white light source as the first light source, and with a blue light source as the second light source, which is suitable for fluorescence observation.

The cell collection device of the present invention preferably includes cell-aspirating means that includes an electro-osmotic pump. It is preferred that the cell-aspirating means comprise a capillary that aspirates cells at the distal end side of the electro-osmotic pump, and a siphon tube in communication with the culture medium of the cells at the proximal end side of the electro-osmotic pump. In this case, during use, the siphon tube is immersed in the culture medium of the cells to fill the inside of the capillary, the electro-osmotic pump, and the siphon tube with the culture medium. This makes it possible to uniform liquid pressure inside the entire capillary, electro-osmotic pump, and siphon tube, and allows accurately activating the electro-osmotic pump only by a voltage applied to the electro-osmotic pump without being affected by the liquid, thereby ensuring reproducible precise aspirating and discharging operations.

It is preferred that the cell collection device of the present invention comprise a two-dimensional stage on which the vessel containing the cells is placed, a first driving system for driving the first optical system and the cell-aspirating means, and a second driving system for driving the two-dimensional stage. It is preferred that the first driving system and the second driving system be independent from one another, and that the resolution of the first driving system be smaller than that of the second driving system. This configuration allows quickly moving the cell-aspirating means (as described in an example described below, cells that have been aspirated by the cell-aspirating means are transferred to a cell-harboring vessel, such as wells of a microplate) and a low resolution optical system by the low resolution driving system, which need to be moved for a large distance, and moving the two-dimensional stage by the high resolution driving system which needs to be precisely moved, thereby ensuring significantly increasing work efficiency of the cell collection.

The present invention will be specifically described in detail below based on the examples. However, the present invention is not limited to the following examples. Examples

FIG. 1 is a diagram illustrating an appearance of the cell collection device of the present invention. A cell collection driving system X-axis shaft 102 is disposed on a main plate 101, and a cell collection driving system X-axis motor 103 serving as a power source for rotating the X-axis shaft is coupled to a terminal of the cell collection driving system X-axis shaft 102. The cell collection driving system X-axis shaft 102 is fitted to a screw hole of an X-axis block 104 and further the lower portion of the X-axis block 104 is fitted to an X-axis guide groove 105, and thus the X-axis block 104 performs translatory movement by rotation of the cell collection driving system X-axis shaft 102. An X-axis guide bar (not shown) is disposed parallel to and opposed to the cell collection driving system X-axis shaft 102, an X-axis guide block 106 is attached to the X-axis guide bar in a manner paired with the X-axis block 104, and the translatory movement is performed along the guide bar. By coupling these two blocks, the translatory movement can be performed more stably. A cell collection driving system Y-axis shaft 107 is used for coupling the X-axis block 104 and the X-axis guide block 106. This allows compounding control of a Y-axis block 108 that allows translatory movement in a Y direction and control of the X-axis, and allows free movement in the two X and Y directions. Furthermore, a cell collection driving system Z-axis shaft 109 is coupled to the upper surface of the Y-axis block 108, and a Z-axis block 110 performs translatory movement in the vertical direction with respect to the XY plane. Thus, the position of the Z-axis block 110 can be freely controlled three dimensionally.

A small plate 111 is attached to the Z-axis block 110, a low magnification optical system lens barrel 112 and an electro-osmotic pump guide 113 are attached to the plate, and these can be moved up and down together with the Z-axis block 110. Furthermore, a low magnification objective lens 114 is connected to below the low magnification optical system lens barrel 112, and a low magnification photographic camera 115 is connected to above the low magnification optical system lens barrel 112. A low magnification light source 116 is connected to the side of the low magnification optical system lens barrel 112, and photographing with coaxial illumination is possible. On the other hand, an electro-osmotic pump 117 to aspirate cells is coupled to the electro-osmotic pump guide 113, allowing adjustment of a position and an angle. Also, a power cable 118 that supplies electric power to the electro-osmotic pump 117 is connected to a control box 119. A voltage control board is housed in the control box 119 and can output any voltage upon an instruction from, for example, a control computer. In addition, for example, all of a camera, a light source, and a drive motor of each axis are connected to the control box 119, and each control is performed as necessary.

Further, on the main plate 101, a two-dimensional cell seeking stage 120 is installed at a position below a basic position of the low magnification objective lens 114. Precision axis shafts that allow driving in the X direction and the Y direction and a precision motor that rotates these shafts for displacement are connected to the two-dimensional cell seeking stage 120. The screw pitch of the precision axis shafts is approximately from 0.1 mm to 1 mm, and a rotational resolution of the precision motor is approximately from 1/1024 to 1/4096, and thus extremely high precise position control with driving resolution of 2 micrometers or less is possible. The precision motor is also connected to the control box 119 with a cable, and can be moved to any position upon an instruction from, for example, the control computer. As described above, while an automatic stage that can be driven at high resolution and in wide range is extremely expensive, since the wide range driving is separated as the cell collection driving system in the present invention, it is sufficient that the two-dimensional cell seeking stage 120 can be driven approximately from 15 mm squares to 35 mm squares. On the two-dimensional cell seeking stage 120, a cell culture vessel 121 is installed also at a position below the basic position of the low magnification objective lens 114. As the cell culture vessel 121, a variety of vessels can be used, but to reduce instability of an operation due to evaporation of cell suspension, a dish vessel, which has a deep bottom and is provided with a cover, is easy to handle. A cell culture vessel cover 122 has an opening having a shape corresponding to the drive range of the two-dimensional cell seeking stage 120 at the center, and a capillary 123, which is connected to the distal end of the electro-osmotic pump 117, enters the inside of the cell culture vessel 121 through this opening to aspirate the cells. The up/down movement of the electro-osmotic pump 117 required at this time is performed by rotation of the cell collection driving system Z-axis shaft 109.

Note that a siphon tube 124 is connected to the terminal portion on the side opposite to the capillary side of the electro-osmotic pump 117. The distal end of the siphon tube 124 is immersed in a cell suspension through a hole provided at the end of the cell culture vessel cover 122. This is a unique configuration by the inventors of the present application. By filling the entire electro-osmotic pump 117 including the capillary 123 and the siphon tube 124 with liquid, the same water pressure acts on the distal end of the capillary 123 and the distal end of the electro-osmotic pump 117, and therefore a liquid flow does not occur at all unless a voltage is applied to the electro-osmotic pump 117 and a stable operation can be achieved. When such a configuration is not used, since the liquid surface position of the terminal portion of the electro-osmotic pump 117 changes due to aspirating and discharging of the cell suspension, the water pressure acting on the capillary 123 also changes. Thus, a liquid flow occurs due to a factor other than the voltage, and therefore especially control of an extremely trace amount of liquid flow required to operate the cells is extremely difficult.

After appropriately aspirating the cells by voltage control of the electro-osmotic pump 117, the electro-osmotic pump 117 is picked up from the cell culture vessel 121, the cell collection driving system X-axis shaft 102 and the cell collection driving system Y-axis shaft 107 are driven, the capillaries 123 is inserted into the target position among a large number of wells provided in a cell collection vessel 125 disposed on the main plate 101 to discharge the cells. The feature of the electro-osmotic pump is that the control of the extremely trace amount of liquid flow required to aspirate and discharge cells can be highly accurately performed by only control of the voltage. After the discharging of cells is completed, the capillary 123 is again returned to the cell culture vessel, and the next cell is sought.

The cells can be sought only with the image of the low magnification photographic camera 115 connected to the low magnification optical system. However, to confirm that the cell is the cell desired to be collected by the user more accurately, it is preferred that the high magnification optical system be used for ensuring observation of the form and the surface state of the cell. To perform this quickly, in the cell collection device of the present example, the high magnification optical system is disposed on the bottom surface side of the cell culture vessel 121. Similar to the low magnification optical system, the high magnification optical system includes a high magnification optical system lens barrel 126, a high magnification photographic camera 127, and a high magnification light source 128. The high magnification photographic camera 127 and the high magnification light source 128 are connected to the control box 119, and an image can be taken in and displayed immediately upon an instruction from the control computer. The reason that the high magnification optical system is disposed on the bottom surface side of the cell culture vessel is that a depth of field of the high magnification optical system is generally small and in the case of photographing from the upper side, a change in the liquid surface is likely to be affected. An example of the combination of the magnifications of the low magnification objective lens and the high magnification objective lens is x5 and x20, but the combination may be changed depending on the size and the shape of cells desired to be collected. In any case, limiting the magnifications to two types allows a configuration in which the cell culture vessel as illustrated in FIG. 1 is sandwiched from the upper side and the lower side for observation.

The first advantage of this system is that the center of the field of view can always be the same by the fact that the optical axes of the two optical systems are adjusted to be coincident. Therefore, by adjusting the position of the capillary 123 so as to aspirate the cells at the center of the field of view, the cell is efficiently sought in a wide field of view using the low magnification optical system, the cell desired to be collected is moved to the center of the field of view, switching to an image of the high magnification photographic camera is performed immediately, and thus details of the cells can be confirmed. The second advantage of this system is that the time to switch the magnification is not required. A general microscope requires an operation to physically move an objective lens to switch the magnification, so this operation inevitably requires several seconds. However, with the system of the cell collection device of the present invention, switching of the magnification is completed only by switching of the camera by a software. Thus, switching can be instantly performed. When only a single cell is collected, this time does not give a significant effect so much, but in an experiment in which cells are collected one by one to a 96-well collection vessel, there is a difference in overall processing time by minutes. Since there is a circumstance that the collected cells are desired to be moved to an environment appropriate for culture or analysis as soon as possible, the difference in time possibly largely affects the accuracy of experiment.

In addition, the third advantage of this system has a feature that the light source used to photograph cells can be switched. For example, to seek for cells in the low magnification optical system, the white light source for confirming a bright field image of the cell is usually employed as the low magnification light source 116, but, for example, a blue light source for detecting fluorescence of the cell can be employed as the high magnification light source 128 and used to seek for the cell at the low magnification. This is possible because the optical axes of the two optical systems are coincident, and it is very convenient when only cells that emit fluorescence are desired to be collected from a cell population. Similar to this, when the cells are observed in detail in the high magnification optical system, the use of the low magnification light source 116 is possible. Generally, to observe a bright field image of cells, the use of transmitted illumination condensed more than coaxial illumination allows obtaining a clearer cell image, and thus this illumination method is suitable for the purpose. The illumination can be switched instantly via the control box 119 by a software, and therefore photographing at the same magnification can be easily performed with the two types of illuminations depending on the case. Examples of such an application include that first the cell is sought using highly sensitive fluorescence and then switching to a bright field image is performed to confirm that the cell is actually present, or it is prevented that a cell not emitting fluorescence present nearby is erroneously aspirated. These applications are extremely important in the purpose that only a single cell is desired to be reliably collected.

After confirmation of the cells to be collected in this way, it is necessary to perform a series of operations, aspirating and holding by the electro-osmotic pump, movement to a predetermined well position in the cell collection vessel, discharging of the cells to wells, and return to the cell seeking position. First, as described above, since the operation of the electro-osmotic pump is stabilized by the use of the siphon tube 124, the cells are automatically aspirated, held, and discharged by outputting preset voltages in order. Also, since the configuration allows three-dimensional position control, when the shape of the cell collection vessel is designated in advance, movement to the predetermined position is also automatically performed. Accordingly, the user only needs to confirm and designate the target cell, and, for example, an operation of pressing a button once allows easily and quickly performing the subsequent collection operation.

A PC (not shown) is connected to the control box 119. In operation, the cells are collected primarily by mouse operation while a monitor (not shown) connected to this PC is seen. When the user visually confirms the cell on the monitor, finds that it is the collection target, and clicks the cell, a monitor coordinate (click coordinate) and a machine coordinate are converted by a software and displacement is automatically performed such that the target cell comes to the "target position."

A calibration work needs be performed in advance to precisely convert the monitor coordinate and the machine coordinate. Specifically, a calibration mode is started and a mark, such as a cell, on a top left of a screen is clicked. Next, a stage is manually moved with, for example, an arrow key such that the mark comes to the lower right (the diagonal position of the initial position) as much as possible and then the mark is clicked again. In this way, since the displacement of the monitor coordinate is found from the two click positions, and the amount of displacement of the stage is found from how many times that the movement command has been transmitted by the manual operation, a coefficient required for conversion of the monitor coordinate and the machine coordinate is determined.

The "target position" designates that the cells are brought to what extent anterior position with respect to the distal end of the capillary on the monitor. When the target position is too far, the aspirating force cannot act and the cells cannot be aspirated, and when the target position is too close, the cells are caught on a step difference due to the thickness of the wall of the capillary and also cannot be aspirated. This is determined experimentally, and it has been found that a value roughly around 50 µm is satisfactory. The target position is also determined by switching the software to a target position designation mode in advance, and then clicking a position approximately 50 µm of the distal end of the capillary on the monitor. When it is determined, a "+" mark is displayed on the monitor.

The above-described pre-setting basically only needs to be performed once. After the setting, the user clicks the cell desired to be taken and visually confirms that the cell has moved to the position of the "+" mark. When there is no problem, clicking the "Aspirate" button applies a voltage to the electro-osmotic pump to aspirate the cell. When it is confirmed that the aspirating has been performed without problems, clicking the "Discharge to the Plate" button automatically performs a step of moving to a well of the plate and applying a discharge voltage.

Note that the control using the PC and the software itself described above are widely used for the known cell collection devices, and can be easily performed by those skilled in the art.

The features of the cell collection device of the present example described above are given below.
(1) A high resolution driving system for seeking cells in a cell culture vessel is separated from a wide range driving system for transferring aspirated cells to a collection vessel. Note that, since "separated" here means ensuring functional separation and independence for driving, the high resolution driving system may be mounted on the wide range driving system. That is, for example, a configuration in which Z-axis and Y-axis units are not coupled, the optical system and the cell collection system only move up and down, and instead, the plate is attached to the two-dimensional wide range driving system of combining the X-axis and the Y-axis, and further the high resolution driving system is mounted thereon is also possible. In this case, it is possible that first, when the movement between the cell culture vessel and the collection vessel is performed roughly (note that this means rougher than the cell size of approximately 10 µm and allows control at the resolution of approximately 20 µm) in the wide range driving system, the collection vessel system is moved upward, and the cell collection system is moved down, and further fine position adjustment is performed in the high resolution driving system on the targeted cell.
(2) Means for aspirating and/or discharging cells is the electro-osmotic pump that is continuously filled with liquid from a cell-aspirating instrument connected to the distal end of the electro-osmotic pump to a tube connected to a terminal of the electro-osmotic pump, and both of them are immersed in the inside of the cell suspension at the stage of aspirating cells. Note that the position in which the tube connected to the terminal of the electro-osmotic pump is immersed is desirably outside the cell seeking range, and may be a region where no cell is present as long as the cell suspension and the liquid surface are shared in the structure.
(3) The low magnification optical system is disposed above the cell culture vessel, the high magnification optical system is disposed below the cell culture vessel, and the optical axes of the optical systems are coincident. Furthermore, it is possible to observe and photograph cells in the high magnification optical system with illumination light incident from the low magnification optical system side, and/or it is possible to observe and photograph cells in the low magnification optical system with illumination light incident from the high magnification optical system side. Note that "low magnification" here means the use of one with approximately x4 to x10 as the objective lens, and "high magnification" here means the use of one with approximately x20 to x40 as the objective lens. A magnification variable lens may be used as necessary.

### Reference Signs List

- 101: Main plate
- 102: Cell collection driving system X-axis shaft
- 103: Cell collection driving system X-axis motor
- 104: X-axis block
- 105: X-axis guide groove
- 106: X-axis guide block
- 107: Cell collection driving system Y-axis shaft
- 108: Y-axis block
- 109: Cell collection driving system Z-axis shaft
- 110: Z-axis block
- 111: Small plate
- 112: Low magnification optical system lens barrel
- 113: Electro-osmotic pump guide
- 114: Low magnification objective lens
- 115: Low magnification photographic camera
- 116: Low magnification light source
- 117: Electro-osmotic pump
- 118: Power cable
- 119: Control box
- 120: Two-dimensional cell seeking stage
- 121: Cell culture vessel
- 122: Cell culture vessel cover
- 123: Capillary
- 124: Siphon tube
- 125: Cell collection vessel
- 126: High magnification optical system lens barrel
- 127: High magnification photographic camera
- 128: High magnification light source

## Claims

1. A cell collection device comprising an optical system to observe cells contained in a transparent vessel under magnification, wherein said optical system comprises a first optical system to observe said cells from upper side and a second optical system to observe said cells from lower side, said first optical system and said second optical system have different magnifications, and wherein the optical axis of said first optical system and the optical axis of said second optical system are coincident.

2. The cell collection device of claim 1, wherein said first optical system has a magnification lower than that of the second optical system.

3. The cell collection device of claim 2, wherein the magnification of said first optical system is from x4 to x10, and the magnification of the second optical system is from x20 to x40.

4. The cell collection device of any one of claims 1 to 3, wherein said first optical system comprises a first light source and said second optical system comprises a second light source, and wherein said first light source enables observation with said first optical system and said second optical system, and said second light source enables observation with said second optical system and said first optical system.

5. The cell collection device of claim 4, wherein said first light source and said second light source are of different types.

6. The cell collection device of any one of claims 1 to 5, comprising a cell-aspirating means including an electro-osmotic pump, wherein said cell-aspirating means comprises a capillary to aspirate a cell at the distal end of said electro-osmotic pump, and a siphon tube communicating with a culture medium of said cells at the proximal end of said electro-osmotic pump.

7. The cell collection device of claim 6, comprising a two-dimensional stage for placing said vessel containing said cells, a first driving system for driving said first optical system and said cell-aspirating means, and a second driving system for driving said two-dimensional stage, wherein said first driving system and said second driving system are independent, and said first driving system has a resolution lower than that of said second driving system.

8. Use of the cell collection device of any one of claims 1 to 7, for collecting a single cell.

9. A method of collecting a single cell, said method comprising collecting a single cell from cells contained in said vessel of said cell collection device of any one of claims 1 to 7.
